# EUROPEAN PATENT SPECIFICATION

(11) **EP 0 873 314 B1**
(45) Date of publication and mention of the grant of the patent: **10.11.1999**
(21) Application number: 96930999.6
(22) Date of filing: 02.09.1996
(51) Int. Cl.: C07D 213/61

(54) **NITRATION OF PYRIDINE-2,6-DIAMINES**
NITRIERUNG VON PYRIDIN-2,6-DIAMINE
NITRIFICATION DE PYRIDINE-2,6-DIAMINES

(30) Priority: 19.09.1995 NL 1001238
(43) Date of publication of application: 28.10.1998
(73) Proprietor: Akzo Nobel N.V., 6824 BM Arnhem (NL)
(72) Inventor: SIKKEMA, Doetze, Jacob, NL-6861 AT Oosterbeek (NL)
(74) Representative: Schalkwijk, Pieter Cornelis
(86) International application number: EP9603841
(87) International publication number: WO9711058

(56) References cited:
- DE-A- 3 920 336
- US-A- 4 310 671
- JOURNAL OF HETEROCYCLIC CHEMISTRY, vol. 32, no. 2, March 1995, PROVO US, pages 585-590, XP002003030 H. RITTER ET AL.: "Synthesis and reactions of dinitrated amino and diaminopyridines" cited in the application
- JOURNAL OF HETEROCYCLIC CHEMISTRY, vol. 8, 1971, PROVO US, pages 841-843, XP002003031 R.L. WILLIAMS ET AL.: "The chemistry of aryltetraamines. II. The synthesis of 2,3,5,6-tetraaminopyridine." cited in the application

## Description

The invention pertains to a process for preparing nitrated pyridine-2,6-diamines in which a pyridine-2,6-diamine is contacted with a mixture of nitric acid and sulphuric acid. By the term pyridine-2,6-diamines are meant, 2,6-diaminopyridine (DAP) and derivative compounds with substitution in the pyridine ring of either or both amino groups and/or carbon atom no. 4 (C-4).

The resulting 2,6-diamino-3,5-dinitropyridine (DADNP) is a suitable starting material for the manufacture of monomers for rigid rod polymers such as described in WO 94/25506 (reduction of the two nitro groups produces the desired tetra-amine). DADNP can also be used as an insensitive (safe) explosive and as a multifunctional organic reagent.

The nitration of DAP by having it react with a mixture of nitric acid and sulphuric acid is known from DE 39 20 336. The drawback to this process is that it gives a DADNP yield of not more than 50% of theory.

Another disclosure of the nitration of DAP by reacting it with a mixture of sulphuric acid and nitric acid is Williams et al., *J.Heterocyclic Chem.,* **8**, 841-843 (1971). Williams discloses the use of concentrated sulphuric acid and a small amount of 90% nitric acid. Yield data other than for crude product are not given.

Further background art is Ritter et al., *J.Heterocyclic Chem.,* **32**, 585 (1995), which mentions the nitration of DAP using nitric acid and indicates the occurrence of by-product formation.

In view of the above it is desired to come up with a DAP nitration process which will produce a higher yield and so offer greater economic advantage as well as making for lower amounts of waste material.

The invention therefore has for its object to provide an economic process by which pyridine diamines can be nitrated with good yield of neat product, without substantial by-products. To this end the invention consists of a process of the known type mentioned in the opening paragraph in which the sulphuric acid used is fuming sulphuric acid. By fuming sulphuric acid is meant according to the invention: over 100%-sulphuric acid (actually, a solution of sulphur trioxide in 100% sulphuric acid which is also known as oleum).

It should be noted that nitration with a mixture of nitric acid (usually 100% nitric acid) and oleum as such is known. However, it cannot be derived from the background information that this specific mixture is capable of solving the above-described problem. For instance, US 4,310,671 discloses the nitration of 2,6-dichloropyridine in oleum. 2,6-dichloropyridine nitration and DAP nitration cannot be compared. This is clear, int. al., from the fact that with DAP it is a question of dinitration, while 2,6-dichloro-pyridine is subject only to a mono-nitration reaction. Moreover, the 2,6-dichloropyridine nitration is achieved only with difficulty, so it is not surprising that, in general, a powerful nitrating agent is selected. In the case of DAP this option is not at all obvious, given that the dinitration as such proceeds easily and the low yield cannot be explained just like that either.

Further background includes JP Hei-6-220019, from which it is known to nitrate 2-hydroxypyridine with a mixture of fuming nitric acid and concentrated sulphuric acid or oleum, and US 4,560,800, from which it is known to prepare 3,5-dinitrobenzophenone by nitrating benzophenone and a mixture of nitric acid and oleum. In H.H. Licht et al., *Int. Annu. Conf. ICT* (1993), 24th (Energetic Materials: Insensitivity and Environmental Awareness), 6-1/6-8, DADNP is made by nitrating DAP with nitric acid and concentrated sulphuric acid in a number of steps.

The finding that nitration with oleum gives a suprisingly higher yield, well in excess of 90%, may lead one to surmise, without wishing to be bound by this theory, that it was the exclusion of water which led to reduced degradation by hydrolysis of the nitration products which were easy to form as such. In this connection the invention also relates to the nitration reaction of pyridine-2,6-diamines in which a pyridine-2,6-diamine is contacted with a nitrating agent in the form of a mixture of nitric acid and sulphuric acid, but where the presence of water is countered in a different fashion, e.g., by carrying out the reaction in an inherently anhydrous medium (i.e., a medium where the released water is trapped by a compound which is more strongly hygroscopic than the nitration product formed during the reaction), e.g., in the presence of polyphosphoric acid, diphosphorus pentoxide, acetic anhydride, and the like.

The process according to the present invention is preferably carried out in oleum having a sulphur trioxide concentration of 20 to 65%. The sulphur trioxide percentage does not have any real effect on the results. The use of 20%-oleum is preferred for practical reasons. According to the present invention, it is possible to use oleum as commercially available. Alternatively, use may be made of concentrated sulphuric acid with 20% or 65%-oleum added thereto to obtain the desired fuming sulphuric acid concentration. Theoretically, it is possible to start from less concentrated sulphuric acid and to add a larger quantity of sulphur trioxide, but in so far as free sulphur trioxide can be handled at all, it is preferred not to do so for practical reasons. The final concentration of the (fuming) sulphuric acid exceeds 100% and is preferably 104,5 to 114,5%.

Preferably, the nitric acid used in the process according to the invention is concentrated (over 95%), more preferably still, 100% nitric acid is employed. When lower concentrations of nitric acid are used, it is preferred to employ oleum having a high sulphur trioxide content. The favourable effect of this is that on conclusion of the nitration reaction the sulphuric acid is still virtually free of water. The most favourable results are achieved when the fuming sulphuric acid has a concentration of more than 104% (which corresponds to 20%-oleum) and the nitric acid has a concentration of more than 97%.

It is preferred to use not more than 2,1 moles of nitric acid per mole of DAP and to carry out the reaction at a temperature of less than 30°C, preferably between 15° and 25°C. If during treatment the nitrated product precipitates because the intensely acidic medium is diluted with water, it is recommended to keep the contact time between the reaction product in solution and the water as brief as possible (preferably less than 5 seconds), and maintain a low temperature during said contact (preferably less than 25°C).

If so desired, instead of DAP itself there may be used in the process according to the present invention derivative compounds with substitution in the pyridine ring of either or both amino groups and/or carbon atom no. 4 (C-4) with oleum-resistant substituents. General knowledge of the field will enable the skilled person to select suitable substituents, e.g., alkyl, especially lower alkyl such as C1-6 alkyl, halo-alkyl, aralkyl, notably deactivated aralkyl such as nitrotoluenyl.

The invention will be further elucidated with reference to the following unlimitative examples below.

### EXAMPLE 1

In a 3-litre reactor equipped with a rapid stirrer, a large bore neck for solids addition, and a dropping funnel, which is protected against atmospheric moisture entry, 1200 ml of 20%-oleum were cooled to 15°C.
In small portions 2,75 moles (300,1 g) of DAP were added (with quick reclosing of the neck after each addition): this took 90 minutes, and the temperature remained below 25°C, mostly below 20°C. After further stirring for 15 minutes 240 ml of 100%-nitric acid were slowly added in 130 minutes at 18-20°C. In all, about 6 kg of ice were used for cooling during dissolution and nitration. After another 10 minutes of stirring with cooling the somewhat viscous, dark red, clear reaction mixture was stirred into 7 kg of ice; the final temperature of the coagulation mixture was 1.5°C.
The solids were filtered off, washed three times with 1 l of water, and dried for 48 hours at 50°C, 1 mbar. The yield was 2,483 moles, 90,3% DADNP. Pouring out said reaction mixture containing a greater excess of ice gave a better yield still, 94%.

### EXAMPLE 2

The nitration reaction of Example 1 was performed in a 1-litre reactor at 1/3 scale (somewhat shorter times were needed because of the larger specific cooling area) using ice-water cooling. Towards the end of the nitration a 3-litre three-necked flask equipped with a rapid stirrer, a dropping funnel, and a vacuum outlet was charged with 2,5 1 of methanol, which was cooled to 10°C by evacuation and boiling invacuo. Using the dropping funnel, the reaction mixture was stirred into the cold methanol boiling in vacuo in 40 minutes at 9-12°C. The resulting slurry was filtered off and stirred into 1 I of ice/water; the new precipitation was filtered off, washed three times with water, and dried for 30 hours at 50°C and 1 mbar to give a yield of 150,67 g of product, 82,5%. It should be noted that this yield of 82,5% in the synthesis indicates the presence of virtually all of the theoretical amount of nitration product in the reaction mixture. This was confirmed with the aid of a comparable methanol filtrate: the precipitation with methanol of a sample of pure nitrated product (DADNP) in 100% sulphuric acid, collection of the solids at 10°C, and washing with water gave a yield of 83% of the product. Successful recovery of the sulphuric acid and the product in the methanol filtrate thus makes for a quantitative yield in the case of production on a (semi) commercial scale.

## Claims

1. A process for preparing nitrated pyridine-2,6-diamines in which a pyridine-2,6-diamine is contacted with a mixture of nitric acid and sulphuric acid, characterised in that the nitration reaction is carried out in an inherently anhydrous medium.

2. The process according to claim 1 wherein the sulphuric acid is fuming sulphuric acid (oleum).

3. The process according to claim 2, characterised in that the oleum has a sulphur trioxide concentration of 20 to 65%.

4. The process according to claim 2, characterised in that the fuming sulphuric acid has a concentration of more than 104% and the nitric acid has a concentration of more than 97%.

## Patentansprüche

1. Verfahren zur Herstellung von nitrierten Pyridin-2,6-diaminen, wobei ein Pyridin-2,6-diamin mit einer Mischung aus Salpetersäure und Schwefelsäure in Kontakt gebracht wird, dadurch gekennzeichnet, dass die Nitrierungsreaktion in einem inhärent wasserfreien Medium durchgeführt wird.

2. Verfahren nach Anspruch 1, bei dem die Schwefelsäure rauchende Schwefelsäure (Oleum) ist.

3. Verfahren nach Anspruch 2, dadurch gekennzeichnet, dass das Oleum eine Schwefeltrioxid-Konzentration von 20-65% hat.

4. Verfahren nach Anspruch 2, dadurch gekennzeichnet, dass die rauchende Schwefelsäure eine Konzentration von mehr als 104% und die Salpetersäure eine Konzentration von mehr als 97% hat.

## Revendications

1. Procédé pour la préparation de pyridine-2,6-diamines nitrées dans lequel la pyridine-2,6-diamine est mise en contact avec un mélange d'acide nitrique et d'acide sulfurique, caractérisé en ce que la réaction de nitration est effectuée dans un milieu intrinsèquement anhydre.

2. Procédé selon la revendication 1, dans lequel l'acide sulfurique est l'acide sulfurique fumant (oléum).

3. Procédé selon la revendication 2, caractérisé en ce que l'oléum a une concentration de 20 à 65% en trioxyde de soufre.

4. Procédé selon la revendication 2, caractérisé en ce que la concentration de l'acide sulfurique fumant est supérieure à 104% et la concentration de l'acide nitrique est supérieure à 97%.
